# EUROPEAN PATENT SPECIFICATION

(11) **EP 1 000 010 B1**
(45) Date of publication and mention of the grant of the patent: **25.09.2002**
(21) Application number: 98933766.2
(22) Date of filing: 07.07.1998
(51) Int. Cl.: C07C 201/08, C07B 43/02, C07C 303/40, C07C 205/59

(54) **NITRATION PROCESS FOR DIPHENYL ETHERS**
NITRIERUNGSVERFAHREN ZUR HERSTELLUNG VON DIPHENYLETHERN
PROCEDE DE NITRATION POUR ETHERS DIPHENYLIQUES

(30) Priority: 28.07.1997 US 901545
(43) Date of publication of application: 17.05.2000
(73) Proprietor: Syngenta Limited, Guildford Surrey GU2 7YH (GB)
(72) Inventor: ATHERTON, John, Heathcote-Zeneca Huddersfield Work, Huddersfield, West Yorksshire HD2 1FF (GB); BROWN, Stephen, Martin-Zeneca Huddersfield Works, Huddersfield, West Yorkshire HD2 1FF (GB); MUXWORTHY, James, Peter-Zeneca Huddersfield Works, Huddersfield, West Yorkshire HD2 1FF (GB); LENNON, Martin-Zeneca Huddersfield Works, Huddersfield, West Yorkshire HD2 1FF (GB)
(74) Representative: Waterman, John Richard
(86) International application number: PCT/GB98/01991
(87) International publication number: WO 99/005087

(56) References cited:
- WO-A-97/10199
- WO-A-98/19978
- US-A- 4 031 131
- US-A- 4 429 146
- US-A- 4 743 703

## Description

The present invention relates to a process for nitration and, in particular to a process for nitrating diphenyl ether compounds which are useful as herbicides or as intermediates in the synthesis of herbicides.

In WO 9710199 the prior art on the production of certain herbicidal nitro substituted diphenyl ethers is reviewed and it is concluded that none of the prior art methods are particularly satisfactory for use on an industrial scale because they all have the common problem that the processes yield a mixture of the required product and other nitrated isomers. Nitrated isomers of diphenyl ether compounds are often extremely difficult to separate from one another and the quantity of other isomers is often too high for the final product to fulfil the requirements of the regulatory authorities for herbicides. The problem tends to be further exacerbated if the nitrated product is an intermediate in the synthesis of a herbicide rather than the required herbicide itself because the mixture of nitrated compounds means that larger quantities of other reagents must be used than would be necessary if the nitrated isomers could be separated satisfactorily. It is therefore important to ensure that the nitration process is controlled to avoid over nitration and also produces a product mixture containing the highest possible proportion of the desired isomer.

There is disclosed in WO9710199 a process for the preparation of a compound of general formula I: 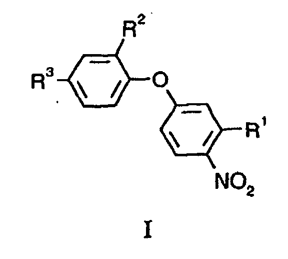 wherein:
R¹ is hydrogen or C₁-C₆ alkyl, C₂-C₆ alkenyl or C₂-C₆ alkynyl, any of which may optionally be substituted with one or more substituents selected from halogen and OH; or COOR⁴, COR⁶, CONR⁴R⁵ or CONHSO₂R⁴;
R⁴ and R⁵ are each independently hydrogen or C₁-C₄ alkyl optionally substituted with one or more halogen atoms;
R⁶ is a halogen atom or a group R⁴;
R² is hydrogen or halo;
R³ is C₁-C₄ alkyl, C₂-C₄ alkenyl or C₂-C₄ alkynyl, any of which may optionally be substituted with one or more halogen atoms; or halo;
   the process comprising reacting a compound of general formula II: 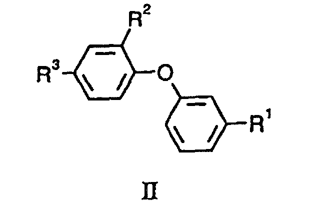
wherein R¹, R² and R³ are as defined for general formula I;
with a nitrating agent comprising nitric acid or a mixture of nitric and sulphuric acids in the presence of an organic solvent and in the presence of acetic anhydride, characterised in that the molar ratio of acetic anhydride to compound of general formula II is from about 1 : 1 to 3 : 1.

These reaction conditions reduced over nitration and therefore gave an improvement in the portion of the required isomer compared to earlier processes.

It has now surprisingly been found that when both nitric and sulphuric acids are used in the process it is possible to further reduce the level of over nitration by adding these acids sequentially to the reaction mixture.

According to the invention there is provided a process for the preparation of a compound of general formula I: 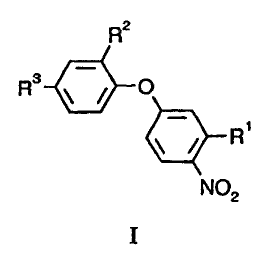 wherein:
R¹ is hydrogen or C₁-C₆ alkyl, C₂-C₆ alkenyl or C₂-C₆ alkynyl, any of which may optionally be substituted with one or more substituents selected from halogen and OH; or COOR⁴, COR⁶, CONR⁴R⁵ or CONHSO₂R⁴;
R⁴ and R⁵ are each independently hydrogen or C₁-C₄ alkyl optionally substituted with one or more halogen atoms;
R⁶ is a halogen atom or a group R⁴;
R² is hydrogen or halo;
R³ is C₁-C₄ alkyl, C₂-C₄ alkenyl or C₂-C₄ alkynyl, any of which may optionally be substituted with one or more halogen atoms; or halo;
   the process comprising reacting a compound of general formula II: 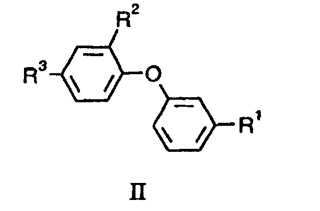
wherein R¹, R² and R³ are as defined for general formula I;
with a nitrating agent comprising nitric and sulphuric acids in the presence of an organic solvent and in the presence of acetic anhydride, the molar ratio of acetic anhydride to compound of general formula II is from 1 : 1 to 3 : 1 characterised in that the acids are added sequentially to the reaction mixture and the organic solvent is perklone.

It has been found particularly advantageous to add the sulphuric acid to a mixture of the compound of formula II and acetic anhydride in the chosen organic solvent, followed by addition of the nitric acid.

The nitric acid is preferably greater than about 90% strength.

The molar ratio of sulphuric acid: compound of general formula II used in the reaction will generally be up to 1.5 : 1, however a ratio of sulphuric acid : compound of general formula II of from 0.1 : 1 to 0.3 : 1 is preferred.

The improved control of nitration results in an increase in yield of the desired product and a reduction in operating costs.

In the context of the present invention, compounds of general formula I are designated 4-nitro isomers. The 2-nitro isomers referred to above have the general formula:

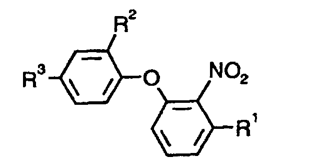

Other mono-nitro isomers which may be produced in the nitration reaction include the 6-nitro isomer:

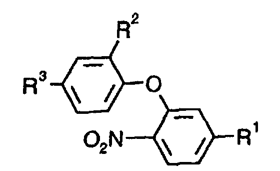

There are also three different dinitro isomers which may be present. Trinitro isomers may also be formed.

In the context of the present invention, the term "C₁-C₆ alkyl" refers to a saturated straight or branched hydrocarbon chain containing from 1 to 6 carbon atoms. Examples include methyl, ethyl, n-propyl, t-butyl, n-pentyl and n-hexyl. The term "C₁-C₄ alkyl" is a subset of C₁-C₆ alkyl and refers to an alkyl group having up to 4 carbon atoms.

The term "C₂-C₆ alkenyl" refers to a straight or branched hydrocarbon chain containing from 2 to 6 carbon atoms and having at least one double bond. Examples include ethenyl, allyl, propenyl and hexenyl. The term "C₂-C₄ alkenyl" is a subset of C₂-C₆ alkenyl and refers to an alkenyl group having up to 4 carbon atoms.

The term "C₂-C₆ alkynyl" refers to a straight or branched hydrocarbon chain containing from 2 to 6 carbon atoms and having at least one triple bond. Examples include ethynyl, propynyl and hexynyl. The term "C₂-C₄ alkynyl" is a subset of C₂-C₆ alkynyl and refers to an alkynyl group having up to 4 carbon atoms.

The term "halogen" refers to fluorine, chlorine, bromine or iodine and the corresponding term "halo" refers to fluoro, chloro, bromo or iodo.

The reaction conditions of the present invention are particularly advantageous since they minimise the over nitration reactions and maximise the amount of the required 4-nitro isomer in the product mixture.

The preferred temperature range for the process of the present invention is from -15 to 15°C, more preferably -10 to 10°C.

The weight ratio of solvent to compound of formula II (including any isomers present) in the process is preferably no greater than 4.25: 1 and it is preferred that the ratio is from 1 : 1 to 2.5 : 1.

Perklone is the preferred solvent.

After the sulphuric acid has been added, the nitric acid is typically added to the reaction mixture over a period of about 30 minutes to 15 hours.

It is often advantageous to add the nitric acid more slowly, for example over a period of from 2 to 15 hours, or, more preferably, 2 to 6 hours.

Although the process of the invention may be used for the preparation of any compound of general formula I, it is especially preferred that R² is chloro and R³ is trifluoromethyl. Particularly preferred compounds of general formula I are those in which R¹ is COOH or CONHSO₂CH₃. These compounds are 5-(2-chloro-a,a,a-trifluoro-4-tolyloxy)-2-nitrobenzoic acid (acifluorfen) and 5-(2-chloro-α,α,α-trifluoro-4-tolyloxy)-N-methanesulphonyl-2-nitrobenzamide (fomesafen), both of which are potent herbicides.

In addition to being a herbicide in its own right, acifluorfen may also serve as an intermediate in the synthesis of fomesafen. The acifluorfen may be converted to the acid chloride which may then be reacted with methane sulphonamide to give fomesafen. Both of these steps may be carried out by conventional methods, for example as set out in EP-A-0003416.

The invention will now be further described by way of the following examples in which the following abbreviations are used:
pph - parts per hundred;
Ac₂O Acetic anhydride;
HPLC - high performance liquid chromatography.

In the comparative examples, the term "mixed acid" refers to a mixture containing 33.6% nitric acid and 66.4% sulphuric acid. The molar quantities given are the moles of nitric acid in the mixture.

### Comparative Example A

### General method for nitration of 3-(2-chloro-α,α,α-trifluoro-4-tolyloxy)benzoic acid in perklone to yield acifluorfen

### a) Nitration

Ac₂O (see Tables I and II for amounts) was added to 3-(2-chloro-α,α,α-trifluoro-4-tolyloxy)benzoic acid (I, R¹ is COOH, R² is chloro, R³ is trifluoromethyl) (20 g, 0.063 mol) in perklone (54 g, 0.635 mol) and the mixture stirred and heated to 40°C to dissolve the starting material. The mixture was then cooled to the appropriate reaction temperature (during which time any crystallisation of the starting material was observed). Mixed acid (13 g, 0.069 mol) was added dropwise over 2 hours and the reaction monitored by HPLC for the completion of the reaction. Further additions of mixed acid were made to reduce the level of starting material to about 1 pph.

### b) Work-Up

The reaction mixture was washed three times as follows:
wash 1 - water (30 ml) was added and the mixture washed at approximately 38°C and the aqueous layer separated;
wash 2 - water (25 ml) was added and the mixture washed at approximately 38°C and the aqueous layer separated;
wash 3 - water (25 ml) was added and the mixture washed at approximately 38°C and the aqueous layer separated.

Water (80 ml) was then added and the mixture heated to 38°C and sodium hydroxide (47% solution, 6.4 g, 0.076 mol) added to basify the mixture to pH 10-11. The mixture was heated to distil off the perklone in order to afford a solution of acifluorfen sodium salt. The solution was cooled to room temperature and transferred with the aid of a minimum amount of water to a bottle in order for the solution to be weighed and analysed.

The results for Experiments 1.1 to 2.3 which were conducted according to the general methods of Comparative Example A are set out in Tables I and II below. In each of these experiments, 20 g crude starting material was used containing 84.3% 3-(2-chloro-α,α,α-trifluoro-4-tolyloxy)benzoic acid. In each of the experiments described in Table I, the amount of solvent used was 54.0 g but for the experiments detailed in Table II, the quantity of solvent was varied. In all the Tables the term "reactant" refers to 3-(2-chloro-α,α,α-trifluoro-4-tolyloxy)benzoic acid.

**TABLE I**

| | | | | | **pph** | | | |
|---|---|---|---|---|---|---|---|---|
| **Exp No.** | **Reaction Temp°C** | **Ac**_{**2**}**O use (mol/mol)** | **HNO**_{**3**} **use (mol/mol)** | **Product Yield %** | **2'-nitro** | **6'-nitro** | **Total dinitro** | **Reactant** |
| 1.1 | -10 | 1.40 | 1.20 | 82.1 | 5.28 | 2.54 | 4.50 | 9.16 |
| 1.2 | 0 | 1.40 | 1.16 | 84.7 | 6.36 | 3.06 | 7.60 | 3.39 |
| 1.3 | 10 | 1.40 | 1.22 | 82.1 | 7.58 | 3.68 | 6.05 | 2.88 |
| 1.4 | -10 | 2.00 | 1.18 | 87.5 | 5.46 | 2.82 | 5.15 | 3.25 |
| 1.5 | 0 | 2.00 | 1.20 | 85.3 | 7.03 | 3.61 | 5.98 | 1.86 |
| 1.6 | 10 | 2.00 | 1.27 | 84.5 | 7.56 | 3.89 | 7.33 | 1.46 |
| 1.7 | -10 | 3.00 | 1.24 | 85.9 | 7.01 | 3.46 | 1.34 | 1.08 |
| 1.8 | 0 | 3.00 | 1.21 | 85.9 | 6.29 | 3.66 | 7.00 | 1.07 |
| 1.9 | 10 | 3.00 | 1.16 | 82.2 | 8.86 | 4.83 | 3.91 | 0.00 |
| 1.10 | 0 | 1.40 | 1.13 | 80.0 | 6.35 | 3.33 | 4.15 | 9.37 |
| 1.11 | 10 | 1.40 | 1.13 | 83.0 | 7.80 | 4.02 | 5.01 | 3.67 |
| 1.12 | 0-5 | 3.00 | 1.20 | 85.4 | 8.07 | 4.43 | 5.89 | 0.00 |
| 1.13 | 0-5 | 3.00 | 1.20 | 84.6 | 7.94 | 4.43 | 5.25 | 0.00 |

**TABLE II**

| | | | | | | **pph** | | | |
|---|---|---|---|---|---|---|---|---|---|
| **Exp No.** | **Solvent usage (g)** | **Reaction Temp°C** | **Ac**_{**2**}**O use (mol/mol)** | **HNO**_{**3**} **use (mol/mol)** | **Product Yield %** | **2'-nitro** | **6'-nitro** | **Total dinitro** | **Reactant** |
| 2.1 | **27.0** | -10 | 2.00 | 1.27 | 85.7 | 5.77 | 2.97 | 5.52 | 2.07 |
| 2.2 | **54.0** | -10 | 2.00 | 1.18 | 87.5 | 5.46 | 2.82 | 5.15 | 3.25 |
| 2.3 | **100.0** | -10 | 2.00 | 1.27 | 84.9 | 5.28 | 2.85 | 6.07 | 2.45 |

### Example 1

### Nitration of 3-(2-chloro-α,α,α-trifluoro-4-tolyloxy)benzoic acid sodium salt

3-(2-Chloro-α,α,α-trifluoro-4-tolyloxy) benzoic acid sodium salt (550 g, of a 36.4% solution) was added to a nitration vessel followed by perklone (734 g). The mixture was heated to 80°C with agitation and 98% sulphuric acid (63 g) added slowly to give a pH of 2. The mixture was allowed to separate and the aqueous layer removed. The solvent layer was washed with water (100g) at 80°C and after separation was dried by azeotropic removal of water up to a batch temperature of 120°C.

The solution of 3-(2-chloro-α,α,α-trifluoro-4-tolyloxy)benzoic acid in perklone was cooled to 60°C and Ac₂O (195 g) and 98% sulphuric acid (63 g) added with stirring. The mixture was cooled to 0°C and 90% nitric acid (53 g) was added at 0°C over 3½ hours with vigorous agitation. The resulting mixture was stirred for I hour at 0°C and then water (190 g) was added. The mixture was heated to 80°C and then allowed to settle. The aqueous layer was removed and the solvent layer washed with water (162 g) at 80°C. Approximately 360 g of perklone was removed by direct distillation and the remainder removed by azeotropic distillation with added water. Once all the perklone had been removed the resulting mixture of molten acifluorfen and water was treated with sodium hydroxide (158 g of a 15% solution) at 90°C. Once the addition was complete the mixture was cooled to 45°C and sodium hydroxide (39 g of a 15% solution) added to give a pH of 7-9. The resulting 40% solution of acifluorfen sodium salt in water was the allowed to cool, yield 85%.

A sample of the product was acidified and the precipitated acifluorfen acid was filtered, washed and dried and gave the following analysis by HPLC using authentic standards for analysis of acifluorfen and the nitrated isomers:

| | |
|---|---|
| acifluorfen | 76.5% |

### Impurities expressed as % weight x 100 / % acifluorfen:

| | |
|---|---|
| 2'-nitro isomer | 7.5 |
| 6'-nitro isomer | 3.9 |
| reactant | 1.5 |
| total dinitro isomers | 1.1 |
| trinitros | 0.4 |

### Example 2

### Nitration of 3-(2-chloro-α,α,α-trifluoro-4-tolyloxy)benzoic acid sodium salt

Further experiments for the nitration of 3-(2-chloro-α,α,α-trifluoro-4-tolyloxy)benzoic acid sodium salt in perklone to yield acifluorfen were performed according to the following general procedure.

A solution of 3-(2-chloro-α,α,α-trifluoro-4-tolyloxy)benzoic acid sodium salt (100% wt, I mol/mol) and perklone (2.26 ml/g of the sodium salt) were charged to a reaction vessel and heated to 80°C. 98% sulphuric acid was then added to adjust the pH to 1.7. The upper aqueous phase was then removed and the residual water removed by distillation. The perklone that co-distilled was returned to the vessel. The mixture was cooled to 30-40°C and acetic anhvdride (1.8-3.0 mol/mol) and 98% sulphuric acid (0.1-1.0 mol/mol) added. The mixture was cooled to 0-5°C and 98% nitric acid (approx 1.21 mol/mol) was added over 2-3 hours while maintaining the temperature of the batch at 0-5°C. The reaction was sampled and checked for completion and additional nitric acid added if required. The mixture was quenched with water and the upper aqueous layer removed at 80°C. The reaction mass was then washed with water at 80°C and the upper aqueous layer removed to leave a crude acifluorfen-perklone mixture. The perklone was distilled azeotropically and the residual perklone and acetic acid removed by steam stripping to afford crude acifluorfen melt which was neutralised at 90°C using 15% aqueous sodium hydroxide to form the acifluorfen salt solution.

The results are shown in Table III

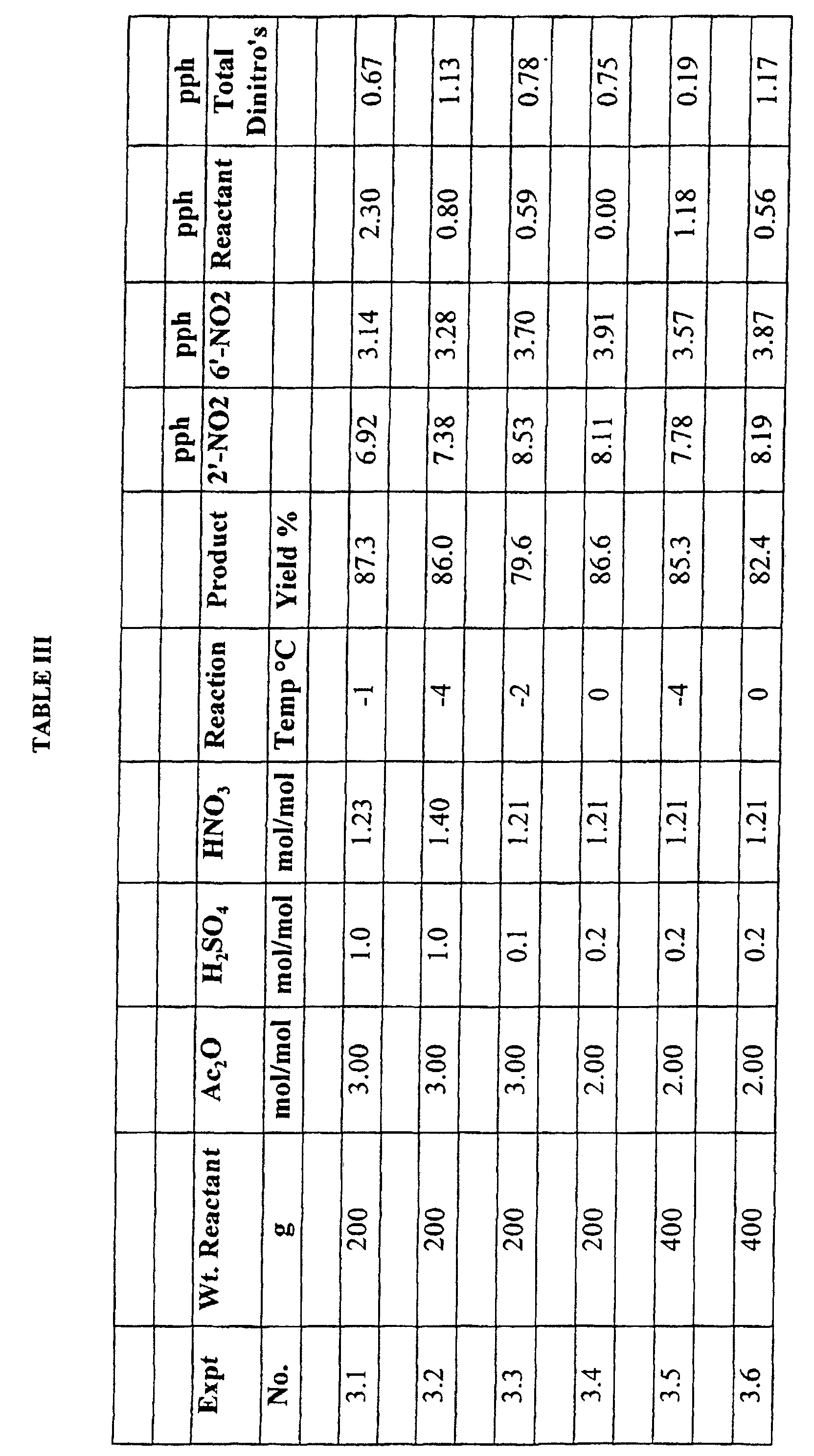

## Claims

1. A process for the preparation of a compound of general formula I: 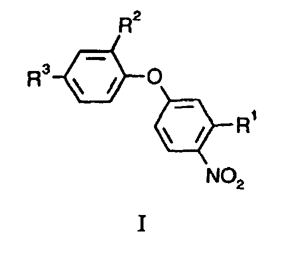 wherein:
R¹ is hydrogen or C₁-C₆ alkyl, C₂-C₆ alkenyl or C₂-C₆ alkynyl, any of which may optionally be substituted with one or more substituents selected from halogen and OH; or COOR⁴, COR^{6,} CONR⁴R⁵ or CONHSO₂R⁴;
R⁴ and R⁵ are each independently hydrogen or C₁-C₄ alkyl optionally substituted with one or more halogen atoms;
R⁶ is a halogen atom or a group R⁴;
R² is hydrogen or halo;
R³ is C₁-C₄ alkyl, C₂-C₄ alkenyl or C₂-C₄ alkynyl, any of which may optionally be substituted with one or more halogen atoms; or halo;
the process comprising reacting a compound of general formula II: 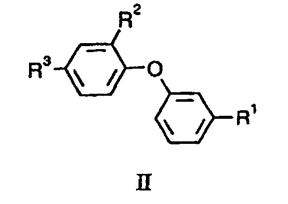
wherein R¹, R² and R³ are as defined for general formula I with a nitrating agent comprising nitric and sulphuric acids in the presence of an organic solvent and in the presence of acetic anhydride, the molar ratio of acetic anhydride to compound of general formula II is from 1 : 1 to 3 : 1 **characterised in that** the acids are added sequentially to the reaction mixture and the organic solvent is perklone.

2. A process as claimed in claim 1, wherein the sulphuric acid is added to a mixture of the compound of formula II and acetic anhydride in the organic solvent, followed by addition of the nitric acid.

3. A process as claimed in claim 1 or claim 2 wherein the nitric acid is greater than about 90% strength.

4. A process as claimed in as claimed in any one of the preceding claims wherein the reaction is performed at a temperature of from -15°C to 15°C.

5. A process as claimed in claim 4, wherein the reaction is performed at a temperature of from -10°C to 10°C.

6. A process as claimed in as claimed in any one of the preceding claims wherein, in the compound of general formula I, R² is chloro and R³ is trifluoromethyl.

7. A process as claimed in as claimed in any one of the preceding claims wherein the compound of general formula I is 5-(2-chloro-α,α,α-trifluoro-4-tolyloxy)-2-nitrobenzoic acid (acifluorfen) or 5-(2-chloro-α,α,α-trifluoro-4-tolyloxy)-N-methanesulphonyl-2-nitrobenzamide (fomesafen).

8. A process as claimed in claim 1, wherein the compound of general formula I is acifluorfen and which further comprises the steps of converting the acifluorfen to its acid chloride and treating the acid chloride with methane sulphonamide to give fomesafen.

## Patentansprüche

1. Verfahren zur Herstellung einer Verbindung der allgemeinen Formel I: 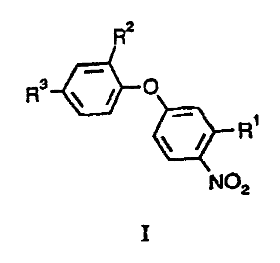 in der
R¹ ein Wasserstoffatom, einen C₁-C₆-Alkylrest, C₂-C₆-Alkenylrest oder C₂-C₆-Alkinylrest, wobei jeder dieser Reste gegebenenfalls mit einem oder mehreren Substituenten, ausgewählt aus Halogenatomen und OH, substituiert sein kann; oder einen Rest COOR⁴, COR⁶, CONR⁴R⁵ oder CONHSO₂R⁴ darstellt;
R⁴ und R⁵ jeweils unabhängig voneinander ein Wasserstoffatom oder einen C₁-C₄-Alkylrest, der gegebenenfalls mit einem oder mehreren Halogenatomen substituiert sein kann, darstellen;
R⁶ ein Halogenatom oder einen Rest R⁴ darstellt;
R² ein Wasserstoff- oder Halogenatom darstellt;
R³ einen C₁-C₄-Alkylrest, C₂-C₄-Alkenylrest oder C₂-C₄-Alkinylrest, wobei jeder dieser Reste gegebenenfalls mit einem oder mehreren Halogenatomen substituiert sein kann; oder ein Halogenatom darstellt;
wobei das Verfahren das Umsetzen einer Verbindung der allgemeinen Formel II: 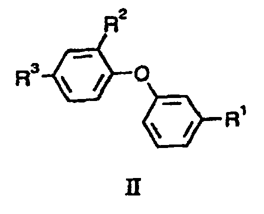 in der R¹, R² und R³ wie für die allgemeine Formel I definiert sind, mit einem Nitrierungsmittel, umfassend Salpetersäure und Schwefelsäure in Gegenwart von einem organischen Lösungsmittel und in Gegenwart von Essigsäureanhydrid, umfasst, wobei das molare Verhältnis von Essigsäureanhydrid zu der Verbindung der allgemeinen Formel II von 1 : 1 bis 3 : 1 beträgt, **dadurch gekennzeichnet, dass** die Säuren nacheinander zu dem Reaktionsgemisch gegeben werden und das organische Lösungsmittel Perklon ist.

2. Verfahren nach Anspruch 1, wobei die Schwefelsäure zu einem Gemisch der Verbindung der Formel II und Essigsäueanhydrid in dem organischen Lösungsmittel gegeben wird, gefolgt von der Zugabe der Salpetersäure.

3. Verfahren nach Anspruch 1 oder Anspruch 2, wobei die Salpetersäure eine Konzentration von größer als 90% hat.

4. Verfahren nach einem der vorstehenden Ansprüche, wobei die Umsetzung bei einer Temperatur von -15°C bis 15°C durchgeführt wird.

5. Verfahren nach Anspruch 4, wobei die Umsetzung bei einer Temperatur von -10°C bis 10°C durchgeführt wird.

6. Verfahren nach einem der vorstehenden Ansprüche, wobei bei der Verbindung der allgemeinen Formel I R² ein Chloratom und R³ eine Trifluormethylgruppe darstellen.

7. Verfahren nach einem der vorstehenden Ansprüche, wobei die Verbindung der allgemeinen Formel I 5-(2-Chlor-α,α,α-trifluor-4-tolyloxy)-2-nitrobenzoesäure (Acifluorfen) oder 5-(2-Chlor-a,a,a-trifluor-4-tolyloxy)-N-methansulfonyl-2-nitrobenzamid (Fomesafen) ist.

8. Verfahren nach Anspruch 1, wobei die Verbindung der allgemeinen Formel I Acifluorfen ist und welches weiterhin die Stufen umfasst, Acifluorfen in sein Säurechlorid zu überführen und das Säurechlorid mit Methansulfonamid zu behandeln, um Fomesafen zu erhalten.

## Revendications

1. Procédé de préparation d'un composé de formule générale I: 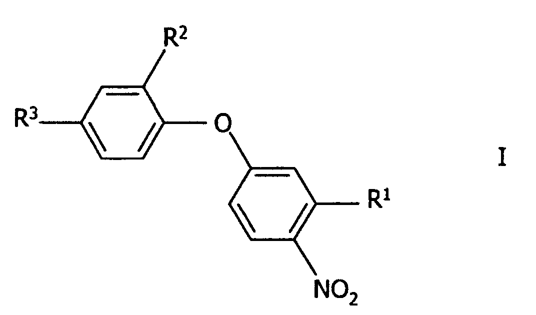 où :
R¹ est l'hydrogène ou alkyle en C₁-C₆, alcényle en C₂-C₆ ou alcynyle en C₂-C₆, parmi lesquels l'un quelconque peut éventuellement être substitué par un ou plusieurs substituants choisis parmi halogène et OH; ou COOR⁴, COR⁶, CONR⁴R⁵ ou CONHSO₂R⁴ ;
R⁴ et R⁵ sont chacun indépendamment l'hydrogène ou alkyle en C₁-C₄ éventuellement substitué par un ou plusieurs atomes d'halogène ;
R⁶ est un atome d'halogène ou un groupe R⁴;
R² est l'hydrogène ou halo ;
R³ est alkyle en C₁-C₄, alcényle en C₂-C₄ ou alcynyle en C₂-C₄, parmi lesquels l'un quelconque peut éventuellement être substitué par un ou plusieurs atomes d'halogène ; ou halo ;
le procédé comprenant la réaction d'un composé de formule générale II : 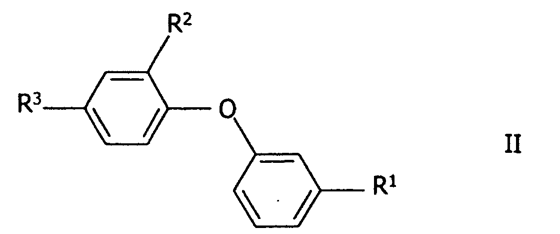
où : R¹, R² et R³ sont définis comme pour la formule générale I avec un agent nitrant comprenant les acides- nitrique et sulfurique en présence d'un solvant organique et en présence d'anhydride acétique, le rapport molaire de l'anhydride acétique au composé de formule générale II est de 1:1 à 3:1, **caractérisé en ce que** les acides sont ajoutés successivement au mélange réactionnel et le solvant organique est la perklone.

2. Procédé selon la revendication 1, où l'acide sulfurique est ajouté à un mélange du composé de formule II et d'anhydride acétique dans le solvant organique, après quoi l'acide nitrique est ajouté.

3. Procédé selon la revendication 1 ou la revendication 2, où la concentration de l'acide nitrique est supérieure à environ 90 %.

4. Procédé selon l'une quelconque des revendications précédentes, où la réaction est conduite à une température de -15°C à 15°C.

5. Procédé selon la revendication 4, où la réaction est conduite à une température de -10°C à 10°C.

6. Procédé selon l'une quelconque des revendications précédentes, où, dans le composé de formule générale I, R² est chloro et R³ est trifluorométhyle.

7. Procédé selon l'une quelconque des revendications précédentes, où le composé de formule générale I est l'acide 5-(2-chloro-α, α, α-trifluoro-4-tolyloxy)-2-nitrobenzoïque (acifluorfen) ou le 5-(2-chloro-α, α, α-trifluoro-4-tolyloxy)-N-méthanesulfonyl-2-nitrobenzamide (fomesafen).

8. Procédé selon la revendication 1, où le composé de formule générale I est l'acifluorfen et qui comprend en outre les étapes de conversion de l'acifluorfen en son chlorure d'acide et de traitement du chlorure d'acide avec le méthanesulfonamide pour former du fomesafen.
